Europäisches Patentamt

European Patent Office

Office européen des brevets

(19)

(11) **EP 1 371 723 A1**

(12) **EUROPEAN PATENT APPLICATION**

(43) Date of publication:
**17.12.2003 Bulletin 2003/51**

(51) Int Cl.⁷: **C12N 7/02, A61K 48/00**

(21) Application number: **02013017.5**

(22) Date of filing: **12.06.2002**

| | |
|---|---|
| (84) Designated Contracting States:<br>**AT BE CH CY DE DK ES FI FR GB GR IE IT LI LU MC NL PT SE TR**<br>Designated Extension States:<br>**AL LT LV MK RO SI** | • **Schneider, Erika, ProCorde GmbH**<br>**82152 Martinsried (DE)**<br>• **Münch, Götz, ProCorde GmbH**<br>**82152 Martinsried (DE)**<br>• **Ungerer, Martin, ProCorde GmbH**<br>**82152 Martinsried (DE)**<br>• **Rosport, Kai, ProCorde GmbH**<br>**82152 Martinsried (DE)** |
| (71) Applicant: **Procorde GmbH**<br>**82152 Martinsried (DE)** | |
| (72) Inventors:<br>• **Kiviniemi, Johanna, ProCorde GmbH**<br>**82152 Martinsried (DE)**<br>• **Baumgartner, Christine, ProCorde GmbH**<br>**82152 Martinsried (DE)** | (74) Representative: **Hartz, Nikolai F., Dr. et al**<br>**Wächtershäuser & Hartz**<br>**Patentanwälte**<br>**Weinstrasse 8**<br>**80333 München (DE)** |

(54) **Process for preparing an adenovirus-containing preparation**

(57)    A process for preparing an adenovirus-containing preparation for *in vivo* somatic gene transfer to the myocardium or endothelium of large animals, comprising the steps of

(i) generating adenovirus in a bioreactor under predetermined conditions for obtaining an adenovirus-containing medium;
(ii) optionally separating of adenovirus from the adenovirus-containing medium of step (i) by filtration to obtain an adenovirus-containing filtrate;
(iii) concentrating the adenovirus-containing medium of step (i) or the adenovirus-containing filtrate of step (ii) to obtain a adenovirus-containing concentrate;
(iv) batch purification of the adenovirus-containing concentrate of step (iii) by a Fast Protein Liquid Chromatography (FPLC) in at least two steps at a pressure of less than 0.3 MPa and a volume of at least 200 ml to obtain an adenovirus-containing batch;
(v) optionally concentrating the adenovirus-containing batch of step (iv) to obtain a concentrated adenovirus-containing batch; and
(vi) optionally dialysis of the adenovirus-containing batch of step (iv) or the concentrated adenovirus-containing batch of step (v),

to obtain an adenovirus-containing preparation.

## Description

### Technical Field

The present invention relates to the identificaton of novel drug targets in the myocardium and in the endothelium of an animal. In particular, the present invention relates to a process for preparing an adenovirus-containing preparation which is suitable for *in vivo* somatic gene transfer particularily into large animals. Moreover, the present invention relates to a method of *in vivo* validation of a target protein in a large animal by somatic gene transfer of an adenovirus into the myocardium or the endothelium of a large non-human animal.

### Background of the Invention

Heart Failure

Congestive heart failure is a chronic disease affecting about 5 Million people in the United States. The five year-mortality rate of patients suffering from congestive heart failure is presently at a level of 50 % whereby specific forms or additional complications lead to drastically increased mortality rates. Congestive heart failure develops when the heart must cope for an extended period of time with an abnormally high demand upon cardiac contractility. An abnormally high demand may be caused by cardiovascular disease such as hypertension and myocardial ischemia, cardiomyopathy or congenital heart disease. Conventional drug therapies of congestive heart failure are directed to an increase of the cardiac output as well as relief of pulmonary congestion and peripheral edema. Cardiac output is conventionally increased by administration of positive inotropic agents stimulating myocardial contractility by enhancing the force and velocity of the myocardial contraction. The oldest and still most important drugs for the treatment of congestive heart failure are based on cardiac glycosides. Cardiac glycosides represent a class of closely related natural products acting directly on the myocardium, specifically, on the membrane-bound $Na^+$ and $K^+$-dependant adenosine triphosphatase. Cardiac glycosides show a complex set of effects including the desired positive inotropic effect and a decrease of the rate of the heart. Due to the very narrow therapeutic range of 1.5 to 2.5, therapy with cardiac glycoside is difficult. In some patients, toxic symptoms are observed at doses required for providing at least partially therapeutic effects. The toxicity of cardiac glycosides comprises both extracardial and cardial effects. Cardiac toxicity produces arrhythmias leading in severe cases to ventricular fibrilations with subsequent systolic arrest and death. Although cardiac glycosides are able to improve the course and the symptoms of congestive heart failure, an improvement of the mortality rate has not been demonstrated with conventional positive inotropic agents. Therefore, the development of alternative therapies for the treatment of congestive heart failure and the identification of agents having positive inotropic effects is highly desirable. The identification of agents having positive inoptropic effects is currently limited by the known biochemical mechanisms on which the contractility of the heart is based. As a consequence, the possibilities for the development of alternative therapies are also extremely limited. Therefore, novel drug targets should be identified to improve the treatment of heart failure.

Artherosclerosis - a disease of the endothelium and the vessel wall

Vessel diseases such as coronary heart disease or stroke currently account for almost 50% of all deaths in the United States and in Europe. Alterations in the vessel wall can induce arterial or venous thrombosis with subsequent disturbance of macro- or microcirculation. In addition to the circulating cells (macrophages, platelets), the endothelium plays a key role not only in the development of vessel disease *per se,* but also for the development of the complications of atherosclerosis. Atherosclerosis is considered a chronic-inflammatory, proliferative tissue response induced by the release of active mediators. Currently existing compounds such as statins have markedly improved the prevention and therapy of atherosclerotic processes. However, these drugs have a number of undesired side effects which limit their use. An important prerequisite for the development of attractive novel pharmacologic compounds is a more detailed understanding of endothelial target proteins and their specific role in the (patho-)physiology of the vessel wall.

Such innovative compounds would offer the potential of highly effective therapies against chronic arteriosclerotic disease as much as for an effective prophylaxis in high risk vascular patients. The development of such novel therapies by using promising vessel wall targets intimately depends on a more detailed molecular understanding and characterization of biochemical and signal transduction pathways in the vessel wall *in vivo.*

In order to arrive at novel therapies for cardiovascular disease, it is necessary to elucidate the biochemical mechanisms underlying the disease. The elucidation of the mechanisms would allow to identify and to influence pharmacological targets selectively. Due to the large number of potential pharmacological targets it is necessary to devise a technology which is useful for the screening and validation of potential targets.

Shortcomings of the present technologies

Current methods to identify novel target proteins:
Out of the approx. 30.000 different proteins in every cell only a small part is functionally relevant at any given moment, depending on the respective pathophysiological condition. Therefore, the information offered by genomic investigation is rather static, whereas all cells - among them cardiomyocytes or endothelial cells - are dynamic units, in which proteins are regulated by disease conditions. For both, myocardium and endothelium, the development of novel therapeutics rather depends on a detailed understanding of protein function ("functional proteomics") in pathophysiological conditions than on genomic investigations. Since the blueprint for all existing proteins, i.e. the genomic DNA sequence, has been identified by the human genome project, the challenge now is to define disease-relevant therapeutic targets on the protein level.

Currently available technologies rely on the investigation of human tissue or animal models both in clinical and in experimental studies. This approach offers the advantage of measuring physiological parameters directly, for instance in response to a therapy with existing drugs. However, this approach does not allow to test the specific molecular effects of novel signalling pathways nor to identify novel drug targets. This is partly due to the fact that existing compounds have a simultaneous impact on several biological pathways and target proteins in the myocardium or in the endothelium. Therefore, a method to unambiguously relate molecular mechanisms to the pathology of vascular and other diseases is unknown in the prior art.

Current technologies rely on the following techniques:

(a) quantitive or qualitative investigation of single proteins or enzymatic activities in patients or different animal models of disease.
(b) genomics and proteomics, i.e. non-directed "screening" of differential mRNA or protein expression in diseased or healthy tissue; analysis by mass spectrometry or microsequencing.
(c) use of transgenic mice to overexpress specific genes in target tissues, among them in the myocardium and the endothelium. Their generation and cross-breeding with disease models, however, takes several months to years.

The results of each experiment according to method (a) must be treated as a single result. Conclusions from the comparison of different experiments are not possible due to the complex differences between the experiments.

Methods according to (b) and (c) are inefficient with regard to the time required to perform the experiments. Moreover, the functional relevance of the vast amounts of data generated by method (b) often remains unclear. This problem still impedes the fast and efficient identification of novel compounds for the treatment of heart and vessel disease.

**Summary of the invention**

It is a problem of the invention to provide novel methods of finding new drug targets in the myocardium and the vessel wall (endothelium), especially for treating heart and vessel disease.

Moreover, it is the problem of the invention to provide a process for preparing an adenovirus-containing preparation which is suitable for *in vivo* somatic gene transfer to the myocardium or endothelium, whereby the adenovirus-containing preparation may be prepared efficiently with regard to time and costs in an amount sufficient for the application to a large animal and with a purity which avoids complications *in vivo,* whereby the method is reproducible and allows a meaningful comparison of multiple *in vivo* somatic gene transfer experiments using a specific adenovirus.

The present invention is directed to identifying and validating new drug target proteins by overexpressing them in the desired target tissues and cells, or by inhibiting them by dominant negative mutant overexpression. Accordingly, target proteins in the myocardium and in the endothelium/the vessel wall can be investigated, and the effect of these interventions on cardiac physiology and on disease endpoints can be studied. The present invention provides for the first time a means to use the myocardium and endothelium routinely for somatic gene transfer *in vivo.* Previously this approach was not available because recombinant virus could not be prepared reproducibly with sufficient sample size at high purity.

In particular, the present invention therefore relates to a method which allows for the adequate preparation and efficient application of recombinant virus *in vivo,* to study the relevance of protein targets in the heart or vessel wall by overexpressing these proteins. Thereby, the invention relates to methods of identifying compounds capable of treating heart failure and/or atherosclerosis.

The present invention provides a process for preparing an adenovirus-containing preparation for *in vivo* somatic gene transfer to the myocardium or endothelium of large animals, comprising the steps of

(i) generating adenovirus in a bioreactor under predetermined conditions for obtaining an adenovirus-containing medium;

(ii) optionally separating of adenovirus from the adenovirus-containing medium of step (i) by filtration to obtain an adenovirus-containing filtrate;

(iii) concentrating the adenovirus-containing medium of step (i) or the adenovirus-containing filtrate of step (ii) to obtain a adenovirus-containing concentrate;

(iv) batch purification of the adenovirus-containing concentrate of step (iii) by a Fast Protein Liquid Chromatography (FPLC) in at least two steps at a pressure of less than 0.3 MPa and a volume of at least 200 ml to obtain an adenovirus-containing batch;

(v) optionally concentrating the adenovirus-containing batch of step (iv) to obtain a concentrated adenovirus-containing batch; and

(vi) optionally dialysis of the adenovirus-containing batch of step (iv) or the concentrated adenovirus-containing batch of step (v),

to obtain an adenovirus-containing preparation.

Preferably, the purification in step (iv) is conducted using columns packed with Q Sepharose XL anion exchange medium. The batch purification comprises at least two steps of Fast Protein Liquid Chromatography (FPLC). It is essential that the pressure is less than 0.3 MPa and the volume is at least 200 ml in order to obtain the required amount and purity of an adenovirus-containing batch for a large animal. The method of the invention provides an adenovirus-containing preparation suitable for somatic gene transfer in an animal larger than a mouse or rat. Surprisingly, it is possible to eliminate contaminants causing fatal complications from an adenovirus-containing mixture by using the method of the invention. The nature of the contaminants and the critical concentration have been unknown in the past and still remain unclear. Therefore, it was conventionally necessary in the past to resort to the complicated cesium chloride density gradient ultracentrifugation method involving time-consuming centrifugation of a small volume for the complete purification of adenovirus-containing mixtures. Although adenovirus could be obtained in high purity and suitable for somatic gene transfer, the conventional methods were unable to provide adenovirus preparations in amounts suitable for animals larger than mice. The adenovirus-containing preparation obtained according to the invention may be used as such. However, it is preferred that subsequent to step (vi) the adenovirus-containing preparation is further processed by concentration or the addition of further components in accordance with common general knowledge in the art and the requirements of the specific application.

The process of the present invention is not limited with regard to the specific adenovirus. However, it is preferred that adenovirus is genetically modified. In particular, the adenovirus may be a recombinant replication deficient adenovirus. The adenovirus preferably contains a gene of interest coding for an expression product associated with cardiovascular disease.

From an industrial point of view, it may be advantageous that in step (i) the adenovirus-containing medium is continuously harvested.

It is preferred that the adenovirus is cultured in step (i) in a bioreactor containing 293 (ATCC CRL 1573) cells which are grown in high glucose DMEM supplemented with 2 percent fetal bovine serum, 2 mM N-acetyl-L-alanyl-L-glutamine, 100 U/ml penicillin and 100 µg/ml streptomycin at 37°C and 5 percent carbon dioxide.

It is preferred that in step (ii) the adenovirus-containing medium is filtered by tangential flow filtration to obtain the adenovirus-containing filtrate. Preferably, the pore size of the filter is in the range of 300 to 1000 nm, more preferably 400 to 600 nm.

The present invention also provides a method of *in vivo* validation of a target protein in a large non-human animal by somatic gene transfer of an adenovirus into the myocardium or the endothelium of a large animal, comprising the steps of

(a) providing an adenovirus-containing preparation according to the method of any one of claims 1 to 12,

(a1) wherein the adenovirus contains a gene of interest which codes for the protein to be validated and which is capable of overexpression of the protein in the myocardium or the endothelium, or

(a2) wherein the adenovirus provides for a dominant negative mutant overexpression of the protein of interest in the myocardium or the endothelium;

(b) providing a test animal as an animal model for heart and/or vessel disease;

(c) administration of the adenovirus-containing preparation to the test animal;

(d) observing the effect of the somatic gene transfer on the animal and selecting the protein as a validated protein when the presence of absence of the protein has a significant impact on the function of the myocardium or the endothelium of the animal;

wherein the animal is sacrificed in the course of the method.

The technology of the invention for efficient gene transfer in vivo allowed to express novel transgenes and candidate proteins in the myocardium and in the vessel wall with high efficiency. These target proteins can thus be assessed for their effects on pathophysiological parameters and disease outcome in relevant animal models of heart and vessel disease.

The animal used is an animal which requires a significant amount of virus for the gene transfer. Specifically, the animal belongs to a species which is suitable as a disease model and which is larger than a mouse or rat. The animal is preferably an animal model for heart failure. Typical animals are rabbits, monkeys, dogs, and pigs. Preferably, the animals are rabbits.

Preferably, in step (c) the adenovirus-containing preparation is administered to the myocardium under high pressure in a high dosage over a short period of time.

It is preferred that in step (iii) the to adenovirus-containing filtrate is concentrated by tangential flow filtration. Preferably, the pore size of the filter is in the range of 10 to 100 nm, more preferably 30 to 60 nm.

**Brief description of the figures**

Figure 1 shows an embodiment of a bioreactor used in the present invention which CelliGen™ Plus packed bed reactor with perfusion system.

Figure 2A shows a graphical representation of glucose and lactate concentrations determined from daily samples of the bioreactor culture in g/l and figure 2B shows a a graphical representation of virus titers in plaque forming units per ml (pfu/ml) of the cell culture supernatant after viral infection of the bioreactor (+) and the oxygen uptake rate measured online by the two dissolved oxygen probes in the set-up (▲). The dashed line in A and B indicates the time of infection.

Figure 3 shows a production scheme for purification of recombinant adenoviruses, using tangential flow filtration for removal of cell debris and for concentration of the bioreactor supernatant and subsequent purification by a two-step anion exchange chromatography protocol.

Figure 4 shows results of a first column chromatography wherein the virus is eluted during the second elution step with a buffer consisting of 52 % elution buffer corresponding to 570 mM NaCl in 20 mM Tris pH 8.

Figure 5 shows results of a second column chromatography wherein the virus is collected to 5 ml fractions during a linear gradient elution step.

Figure 6 shows the results of gel electrophoresis and Coomassie stain wherein it is shown that one chromatographic purification step is not sufficient to achieve a satisfactory purity grade. Lane 1 shows virus after the first chromatography step, lane 2 the same sample further purified and formulated (final dosage form). After only one chromatography step a serum-derived protein, albumin (66 kD) and falsely formed hexon contaminants (about 200 kD), can be detected. Lane 3 shows a $CsCl_2$ gradient purified virus. 1,75 x $10^{10}$ particles were used in each case.

Figure 7 shows results of a Western blot wherein monoclonal antibody specific for the hexon protein (120 kD) in the end product (lane 1) and the CsCl purified control virus (lane 2) can be identified. 3.5 x $10^{10}$ virus particles were used in each case.

Figure 8 shows results of gel electrophoresis wherein the purity of the column chromatography purified virus (end product, lane 1) is identical to the CsCl purified virus (lane 3) 3.5 x $10^{10}$ virus particles were used in each case.

Figure 9 shows the results of a comparison of 2D gel electrophoresis of a conventional preparation (upper panel) and samples generated in bioreactor runs and purified with a method using one column (middle panel) or prepared according to the method of the invention (lower panel).

Figure 10 shows macroscopic (A) and microscopic (B) images of the expression of a recombinant protein in the myocardium of rabbits after trans-coronary gene transfer in vivo 1 x $10^{10}$ pfu of recombinant adenovirus applied via a catheter placed in the coronary artery in rabbits.

Figure 11 shows macroscopic images of the expression of a recombinant protein in the myocardium of rabbits 2 weeks after gene transfer in vivo wherein $4 \times 10^{10}$ pfu of recombinant adenovirus were applied after aortic cross-clamping in rabbits. upper panels (A,C): sham operation, lower panels (B,D): gene transfer of Ad-GFP.

Figure 12 shows microscopic images of the expression of a recombinant protein in the myocardium of rabbits 2 weeks after gene transfer in vivo wherein $4 \times 10^{10}$ pfu of recombinant adenovirus were applied after aortic cross-clamping in rabbits. Left panel: sham operation, right panel: gene transfer of Ad-GFP.

Figure 13 shows a hemodynamic and echocardiographic assessment of heart function in failing rabbits one week after gene transfer with Ad-GFP or Ad-ßARKct in vivo

(A) As a measure of cardiac contractility, the maximum first derivative of left ventricular pressure (+dp/dt max) was measured by intraventricular tip catheters. Isoproterenol-stimulated contractility was significantly better in the animals which expressed ßARKct in their hearts.
(B) Relative decrease in fractional shortening (FS) as assessed by serial echocardiography. The bars show the FS ratios at the final measurements (14 days after the onset of pacing) divided by the measurements before gene transfer (7 days after the onset of pacing) in the same animals.

All measurements were done in 8 animals in triplicates. *$p < 0.05$ vs. GFP.

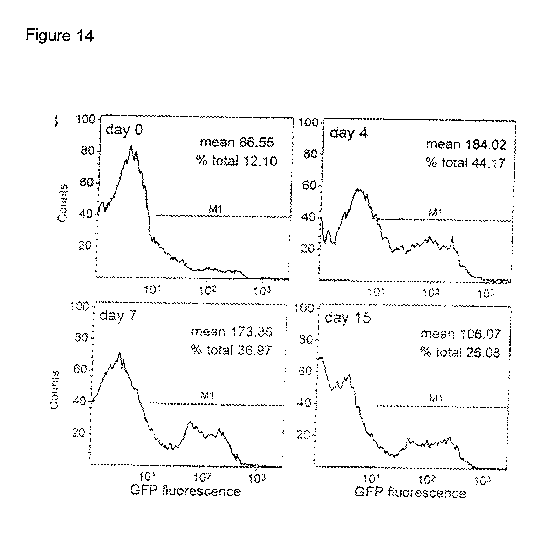

Figure 14 shows transgene expression in LV myocardium after trans-coronary gene transfer in vivo. The time course of the expression of a fluorescent reporter gene was analyzed by FACS in single cardiomyocytes isolated from in vivo infected hearts after the indicated time points. M1 indicates the fluorescence cutoff. 20,000 cells were analysed in each sample. Shown are cumulative data from four animals.

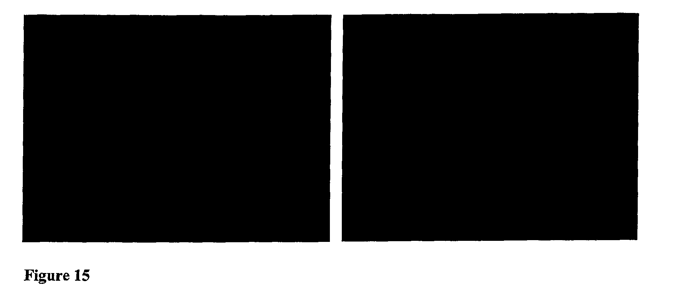

Figure 15 shows expression of a recombinant protein in the endothelium of a carotid artery of rabbits 2 weeks after gene transfer in vivo $1 \times 10^{10}$ pfu of E1-E3 deleted recombinant adenovirus encoding a fluorescent reporter gene were applied after proximal and distal vessel closure in rabbits. left panel: sham operation, right panel: gene transfer of Ad-GFP. Similary results were seen eight weeks after gene transfer of a gutless adenovirus encoding GFP.

**Detailed description of the invention**

The present invention establishes for the first time a highly efficient method for producing large amounts of recombinant adenovirus. It is essential for the present invention that a bioreactor is used for generating adenovirus in step (i). A bioreactor according to the invention is a vessel or apparatus wherein living cells may exhibit their activity under defined conditions. Moreover, the bioreactor must be suitable to provide a sufficient amount of adenovirus. Therefore, the capacity of the bioreactor is preferably at least 0.5 L, more preferably 1 L. Accordingly, the common petri dish or shake flask technology is not suitable for the method of the present invention. The bioreactor may be a bioreactor for suspended cells in submerged cultures or a bioreactor for carrier-bound or immobilized cells in submerged culture. A bioreactor for carrier-bound or immobilized cells is preferred. The bioreactor is preferably a bioreactor for adherent cells since suspended cells provide lower adenovirus yields. The bioreactor may be a tank reactor (height/diameter $\leq 3$) or a column reactor (height/diameter $\geq 3$). Preferably, the bioreactor is supplied with energy. The energy may be supplied mechanically by moved internals, by external liquid pumping (hydraulic mixing), or *via* compressed air (pneumatic mixing). Preferably, the bioreactor is a mechanically stirred bioreactor which may be used for culture of adhering cells. Due to the large amount of virus required according to the invention, the reactor must provide a large amount of surface on which the cells may adhere per volume unit. Preferably, the surface density is at least 5000 $cm^2/L$, more preferably at least 10.000 $cm^2/L$. Examples of the bioreactor types useful for the method of the invention are packed-bed reactors, heat exchanger reactors, hollow-fiber reactors, microsphere reactors, or ceramic monolith reactors. Packed-bed reactors are preferred. Common incubator flasks, roller flasks, multitray reactors or stack-plate reactors are not suitable for the purposes of the invention since the number of cells per volume unit are insufficient. Cultivation of cells in the bioreactor may be carried out by using a batch, repeated batch, repeated fed batch, chemostat or perfusion technique. According to the invention, it is preferred to exactly control the growth conditions of the cells by the use of the perfusion technique.

The cell culture supernatant in the bioreactor used in the method of the invention contains the desired adenovirus. The adenovirus is harvested by removal of supernatant from the bioreactor to provide an adenovirus-containing medium. The adenovirus-containing medium is an aqueous medium further containing a complex mixture of cell fragments, metabolic products and compounds used for culturing cells. The adenovirus-containing medium is unsuitable for use as an adenovirus-containing preparation for *in vivo* somatic gene transfer to the myocardium or endothelium of large animals due to the presence of components leading to complications when administered to a large animal. The nature of the toxic components is unknown.

According to the invention, the adenovirus may be separated from the adenovirus-containing medium of step (i) by filtration to obtain an adenovirus-containing filtrate. Preferably, the separation is carried out by tangential flow filtration. The adenovirus-containing medium or the adenovirus-containing filtrate is concentrated. The adenovirus-containing medium or adenovirus-containing filtrate are unsuitable for use as an adenovirus-containing preparation for *in vivo* somatic gene transfer to the myocardium or endothelium of large animals due to the presence of components leading to complications when administered to a large animal. According to the invention, a batch purification of the adenovirus-containing filtrate or the concentrated adenovirus-containing filtrate of step (iii) Fast Protein Liquid Chromatography (FPLC) comprising at least two steps at a pressure of less than 0.3 MPa and a volume of at least 200 ml to obtain an adenovirus-containing batch is essential. For this purpose, it is preferred to use a liquid chromatography-based double-step purification method run at normal pressure.

The method of the invention allows for the first time to purify sufficiently large sample sizes with high purity and low toxicity.

**Methods**

General Overview

Recombinant replication deficient adenoviruses are generated in a perfusion culture of adhering 293 cells in a 3,5L Celligen™ packed bed bioreactor with a basket impeller system. Viruses are harvested continuously from the cell culture supernatant and subsequently filtrated and concentrated by tangential flow filtration. Concentrates are purified by a two step anion exchange chromatography procedure with Q Sepharose™ XL, dialysed, filtered and analyzed. To this end, a new FPLC-based protocol was established which for the first time allows for efficient purification of large samples of recombiant adenvirus, as derived from bioreactor culture.

It had been shown before that recombinant adenovirus can be produced at larger scale in a bioreactor with semi-adherent batch culture (Wang GGZ, Zhang WY, Julien C. Increased adenoviral vector production. Biopharm July 1999). Other groups had used suspension cultures of producer cells (Gardner et al., Biotechniques 2001; 30: 422-427; Nadeau et al., Biotech Progr 2000; 16:872-884). However, the virus production and purification disclosed in the prior art is unsuitable for *in vivo* gene transfer, in particular to large animals. In addition, some work on the purification of adenovirus by column chromatography had been carried out previously (Huyghe BG, Liou XD, Sutjipto S, Sugarman BJ, Horn MT, Shepard HM, Scandella CJ, Shabram P. Purification of a type 5 recombinant adenovirus encoding human p53 by column chromatography. Hum Gene Ther 1995; 6:1406-1416; patent applications WO96/27677, W097/08298, WO98/00524, WO98/22588, WO 00/40702). Especially, WO98/00524 had described the use of an anion exchange column for preparative purposes using a specific resin (source 15Q). Also in WO 00/40702, a similar method is described, but the focus is rather on establishing a sensitive detection assay for adenovirus using HPLC of small sample volumes, thus not allowing to purify the large samples needed for repeated in vivo experiments in relevant disease models in larger animals. Both protocols allowed for purified viral preparations but had not yielded sufficiently reproducible high amounts of virus for in vivo gene therapy into the heart or vessel wall of mammals.

Cell culture and viruses

293 (ATCC CRL 1573) cells are grown in high glucose (4.5g/L) DMEM (Biochrom AG, Berlin) supplemented with 10% fetal bovine serum (FBS), 2mM N-acetyl-L-alanyl-L-glutamine, 100 U/ml penicillin and 100 µg/ml streptomycin (Biochrom AG, Berlin) at 37°C and 5% $CO_2$. Adenovirus infected cultures are only supplemented with 2% FBS.

Recombinant E1/E3 deleted adenoviruses of serotype 5 carrying one or two transgenes under the control of the strong CMV promotor were generated according to He et al., Proc Natl Acad Sci USA 1998; 95:2509-2514. They are amplified in 293 cells and purified by a two step CsCL gradient centrifugation as described elsewhere (Becker, T. C., Noel, R.J., Coats, W.S., Gomez-Foix, A.M., Alam, T., Gerard, R.D. & Newgard, C.B. (1994) *Methods Cell Biol.* **43**, 161-189). Collected virus is dialysed 4x1h against 40 volumes of dialysis buffer (10mM Tris pH 8, 2mM $MgCl_2$, 5% sucrose) frozen with 10% glycerol and stored in aliquots at -80°C.

Alternatively, recombinant gutless adenoviruses were generated according to standard protocols by using helper vectors containing the genes of interest, an E1/E3-deleted helper adenovirus with a packaging signal flanked by two loxP recognition sites, in cre recombinase-overexpressing HEK 293 cells (Kochanek, Human Gene Therapy 1999; 10:2451-2459). They were further amplified in HEK 293 cells for subsequent rounds by carefully testing for the presence of recombinant first generation virus and for replication-competent virus.

Particle and infectious titer

The number of total DNA containing particles of a purified virus suspension is derived spectrophotometically by Maizel's method (Maizel, JR. J., White, D. & Scharff, M., (1968) *Virology* **36**, 115-125; Shabram, P. W., Giroux, D. D., Goudreau, A. M., Gregory, R. J., Horn, M. T., Huyghe, B. G. Liu, X., Nunnally, M. H., Sugarman, B. J. & Sutjipto, S. (1997) *Human Gene Ther.* **8**, 453-465). Briefly, virus particles are lysed in virion lysis buffer (0.1%SDS, 10mM Tris-HCl pH7.4, 1mM EDTA) and the absorbance at 260 nm is measured. A conversion factor of $1.1 \times 10^{12}$ particles per absorbance unit at 260 nm is used to calculate the particle number.

The infectious particle titer is measured by the TCID50 (tissue culture infectious dose 50%) method. 293 cells are cultivated over-night in 96-well microtiter plates with medium containing 2% FBS and infected with 100µl of 10-fold dilutions of virus solution in 10 replicates each. For fluorescing reporter gene-expressing adenoviruses, the amount of fluorescing reporter gene-positive wells is counted using a fluorescent microscope (485 nm exitation, 515 nm emission) after 72 h, for other viruses, wells showing CPE are counted using a light microscope 7 days after infection. The titer is calculated using the KÄRBER statistical method: for 100µl of dilution, the titer is $T=10^{1+d(S-0.5)}$ with d=Log10 of the dilution and S=the sum of virus positive wells (starting with $10^{-1}$ dilution, 10 of 10 positive=1). To convert the TCID50 titer to the classical pfu titer, the TCID50 is divided by the empirical factor of 19.

Bioreactor set up

A 3,5L working volume Celligen™ Plus bioreactor (New Brunswick Scientific) equipped with a cell retention device (basket) and a Cell lift™ impeller system with a ring sparger (New Brunswick Scientific) is used to culture the 293 cells on a packed bed for production of recombinant adenovirus. Dissolved oxygen and pH control is achieved with the interactive 4-gas-control by sequential addition of air, nitrogen, oxygen and carbon dioxide. Five peristaltic pumps are available on the console for addition or removal of liquids from the culture. AFS BioCommand (New Brunswick Scientific) software on a pentiumII computer is used for data acquisition and process control.

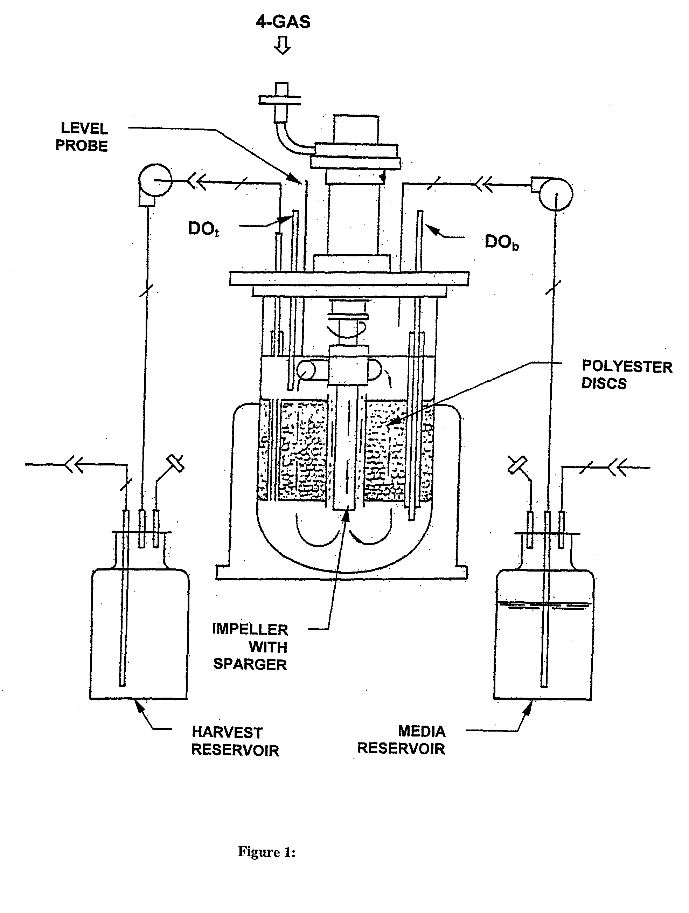

The system (see fig. 1) is set up with one InPro™ 3200 gel pH probe (Mettler Toledo), two InPro™ 6000 dissolved oxygen electrodes (Mettler Toledo). One line for buffer removal after autoclaving, one line for initial medium addition, cell inocculation, infection and medium addition during perfusion, one line for harvest of cell culture supernatant during perfusion, one line for sampling and one line for base addition is connected to the respective ports of the bioreactor's head plate. For all lines, silicon tubings with 5 mm inner diameter and 1.5mm wall thickness are used. The basket is filled with 50-100g of FibraCell™ disks, a three-dimensional polyester matrix with 1200 $cm^2$ area/g. For autoclaving, the vessel is filled with 3,5L phosphate buffered saline and the complete set-up is autoclaved for 30 min at 121°C against a 4L reference, following the manufacturer's instructions. After autoclaving, the buffer is removed and the vessel is filled with 3L of culture medium without phenol-red containing 10% FBS.

Culture set-up

In general, the culture process is divided into two phases: a growth phase with a duration of about 6-7 days and a production phase with a duration ranging from 6 to 8 days, depending on the MOI used for infection of the culture. The following process parameters are used: dissolved oxygen at 50% (calibrated to air saturation, controlled by the top DO electrode), pH at 7.2, temperature at 37°C and stirrer rate at 50 rpm using an airation rate of 21 ml/min. The culture is inocculated with $2 \times 10^4$ 293 cells /g of Fibra Cell™ by using one peristaltic pump of the reactor's console. The culture is maintained in batch-mode for 24-48h following inocculation. Depending on growth parameters, the culture is subsequently switched to perfusion mode using a rate of 3L/d by using two peristaltic pumps of the reactor's console and maintaining the culture volume to 3.5L by adjusting the harvest outlet-line inside the assembly to the desired level. Media is supplied and collected in 10L-20L Flexboy™ containers (Infiltec).

Viral Infection

One day prior to infection of the virus, a medium exchange is initiated with a perfusion rate of 5L/d. Alternatively, the medium is exchanged completely before infection using the buffer outlet line. During the virus production phase, the medium is supplemented with only 2% FBS and lacks phenol red. For adenoviral infection of the culture the perfusion is stopped. During infection, the temperature is lowered to 30°C and the stirrer rate is increased to 100 rpm for one hour following the infusion of $6 \times 10^7$ pfu/g of FibraCell™ in 100 ml culture media via the medium addition line (aproximate MOI=0.1-0.2 pfu/cell).

Perfusion at 3L/d is restarted 4-5 hours after the infection and the culture supernatant is collected at 4°C in 20L Flexboy containers (Infiltec). To complement the pH control by the 4-gas-control, steril-filtered 8% (w/v) bicarbonate ($NaHCO_3$) solution is connected via the base addition line and a peristaltic pump using the <base-addition> function of the Celligen™ console.

Culture parameters

Throughout the process, daily samples are taken via the sampling line and checked for contaminations using a light microscope. The concentrations of D-glucose and L-lactate are measured in the culture medium using a hand-held multianalyzer (SensLab, Leipzig) and immobilized enzyme bio-sensors (SensLab, Leipzig). For metabolite measurements, the medium samples are diluted 100-fold in sample dilution buffer pH 6.8 (SensLab). Of all samples taken during the production phase, additionally the infectious titer is determined using the TCID50 method.

The oxygen uptake rate, which is measured on-line using two dissolved oxygen probes, one located above the packed bed, monitoring and regulating DO in the freshly airated medium and the second located below the packed bed measuring the oxygen depleted medium. The OUR is calculated using the equation:

$$OUR = 60Q * \frac{(Dot-Dob)}{100*Vb} * C$$

Where OUR is the oxygen uptake rate in mol/l/h

Q is the liquid flow rate in L/min

Dot is the percentage of dissolved oxygen of the top DO probe

Dob is the percentage of dissolved oxygen of the bottom probe Vb is the liquid volume of the packed bed in liters

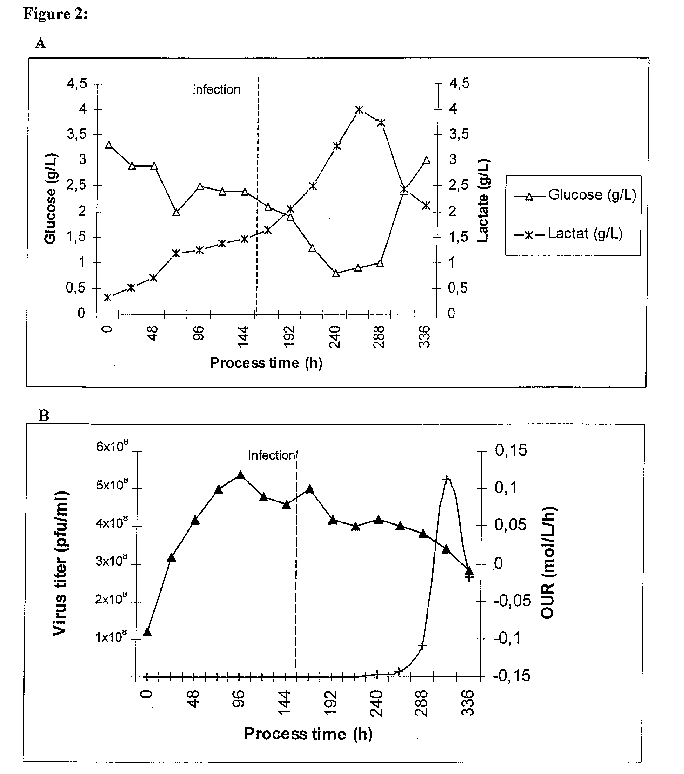

The overall yield of every process is determined by measuring the infectious titer (TCID50) from the collected culture supernatant subsequently to shutting-down the process (see Figure 2)

Down-stream processing

After shut-down, the supernatant is filtered by tangential flow filtration using a TFF MiniKros™ hollow fiber PES module with 4000 cm$^2$ (MembraPure). The pore size of 500 nm allows the adenoviruses (aproximate diameter of 90nm) to pass the membrane. Cells and cell debris are removed from the supernatant in this step. To concentrate the supernantant, a second tangential flow filtration step is added and a MiniKros™ TFF module with 3900 cm$^2$ (MembraPure) with a PS mebrane of 50nm pore size is used and the virus suspension is concentrated to a factor of 10 fold. All modules are prewetted with water following the manufacturer's instructions. The liquid is recirculated through the modules using silicone platinum tubing (Cole Parmer) with 9.6 mm inner diameter and 3.2 mm wall strength and a MasterFlex I/P™ peristaltic pump with a recirculation rate of approximately 10L/min. The filtrate rate (flux) for both modules is aproximately 80L/hm$^2$

Purification of the cell supernatant By FPLC

First column chromatography

An XK 50/20 chromatography column (cat. no 18-1000-71), containing 200 ml of Q Sepharose™ XL anion exchange media (cat. no 17-5072-01) giving rise to a bed height of 10 ± 0.5 cm, is connected to the Äkta Explorer 100 system (all from Amersham BioSciences).

Buffers needed are prepared and filtered through a 0.2 μm filter beforehand. The column is equilibrated with 5 column volumes (cv) of running buffer (20 mM Tris pH 8 + 0.14 m NaCl + 0.1% Tween 20 + 5% sucrose) using a flow rate of 20 ml/min. This flow rate is kept constant throughout the whole run. The sample is loaded onto the equilibrated column using the sample pump P-950. After sample loading step the column is washed with 2 cv of running buffer.

A first elution step is carried out with 2 cv of buffer consisting of 74% running buffer and 26% elution buffer (20 mM Tris pH 8 + 1 M NaCl + 0.1% Tween 20 + 5% sucrose). A second elution step is carried out with a 4 cv linear gradient from 26% to 60% elution buffer while collecting fractions of 10 ml. The fractions giving rise to a peak in the absorbance curve during this second elution step are pooled together. This pool of fractions is diluted with a dilution buffer (20 mM Tris at pH 8) to a concentration of 0.25 M NaCl. After the two elution steps, the column is washed with 1 cv of 100% elution buffer. Re-equilibration of the column with 5 cv of running buffer immediately follows the washing step.

Second column chromatography

XK 16/10 column containing 20 ml source 30 Q anion exchange media (Amersham BioSciences, prepacked, cat. no 17-1275-01), is connected to the Äkta Explorer 100 system. The column is equilibrated with 5 cv of running buffer (20 mM Tris pH 8 + 0.14 m NaCI + 0.1% Tween 20 + 5% sucrose) with a flow rate of 10 ml/min. This flow rate is kept constant throughout the whole run. The sample is loaded onto the equilibrated column using the sample pump P-950. After the sample loading step, the column is washed with 2 cv of running buffer. The virus is eluted with a salt gradient over the concentration range 0- 60% elution buffer over 10 cv. 5 ml fractions are collected beginning at 20% buffer B. After the gradient elution, the column is washed with 1 cv of 100% elution buffer. Re-equilibration of the column follows after the washing step with 5 cv of running buffer.

Formulation

The virus peak fractions are pooled together and formulated to the final dosage form. A bed of 100 ml Sephacryl S-300 matrix (Amersham BioSciences, cat. no 17-0599-01) with a bed height of 5 cm is equilibrated with 5 cv of the formulation buffer (10 mM Tris pH 8 + 0,14 M NaCI + 5% sucrose + 0,1% Tween 20). Thereafter the sample is loaded into the column. Virus is eluted from the column with 1 cv of formulation buffer while collecting 10 ml fractions. Hereby the buffer is exchanged to the formulation buffer and remaining small protein contaminants and nucleotides are removed. Glycerol is added to a final concentration of 10%, virus is filtrated through a 0,2 $\mu$m filter, aliquoted and frozen at - 80°C.

**Analysis**

Gel electrophoresis

The end product is denatured, reduced and run on an SDS polyacrylamide gradient gel (4-20% Tris-HCI gradient gel, Bio-Rad, cat. no 161-1159). Protein bands detected in the gel by Coomassie or silver staining are compared to the identically treated control sample of virus purified with the conventional CsCI gradient method, which is known to have a sufficient purity grade for intravenous application in animals.

Western Blot

The end product is denatured, reduced and run on an SDS polyacrylamide gradient gel (4-20% Tris-HCI gradient gel, Bio-Rad, cat. no 161-1159). Identity of the end product is verified with a monoclonal antibody specific for the structural hexon protein of the adenovirus capsid (Biodesign, cat. C86006M).

Endotoxin assay

Endotoxin concentration in the end product is measured by means of a commercially available endotoxin assay (pyrochrom endprint method, Haemochrom Diagnostica GmbH, cat. no C0120).

Free DNA

Samples of the chromatographically purified end product and the CsCI-purified control virus samples were ultrafiltrated to remove the virus particles. The absorbances of the filtrate solutions were measured with a spectrophotometer at the wavelength of 260 nm.

Two dimensional gel electrophoresis:

The sample is solubilised in strong denaturating buffer containing urea, thiourea, DTT, CHAPS and Pharmalytes. Isoelectric focussing separates proteins towards their isoelectric point. Immobiline pH gradient gels (IPG strips) are used in the pH range 4 - 7. After equilibration in DTT and iodacetamide, the second dimension a SDS - PAGE is performed. Homogenous gels with 13% Acrylamide and 2,7% Bisacrylamide are used. For protein visualisation a maldi compatible silverstaining protocol is carried out.

Enzymatic digestion and sample preparation:

Gel pieces are digested with trypsin and the tryptic peptides are eluted from the gel with acetonitrile / 0.1% TFA according to an optimized protocol. Extracts are purified with $C_{18}$ ZipTips Purified extracts are co-crystallized with matrix (2,5-Dihydroxy benzoic acid) on the target plate.

ALDI-TOF mass spectrometry:

The sample is ionized and desorbed by a pulsed $N_2$-Laser (337 nm). The MALDI-matrix absorbs energy and enhances the ion formation of the analyteby photoexcitation or photoionization of matrix molecules followed by proton transfer to the analyte.

Ions are accelerated into a flight tube by an accelerating voltage of 20-25 kV. The mass-to-charge ratios of ions is determined by measuring the time taken for the ions to travel from the target to the detector.The mass list is exported to a suited database, in which the peptid masses can be identified to the proteins identity.

Gene transfer into cardiomyocytes and into the myocardium

State of the art : gene transfer to the heart in vivo

Some previous studies had investigated gene transfer to the heart and myocardium in vivo. A method for efficient gene transfer into rat hearts had been established before (e.g., Hajjar et al., Proc Natl Acad Sci 1998; 95: 5251-5256; DelMonte et al., Circulation 2001; 104: 1424-1429). In these studies, however, the number of cardiomyocytes that were really infected in vivo had not been assessed, and the approach generally suffers from the problem that human heart failure cannot be simulated sufficiently in rats. Some other efforts had been carried out in larger animals, but they had not resulted in a widely distributed, reproducible gene transfer to all parts of the myocardium in vivo. This insufficient outcome depended on the problem that either gene transfer occurred only locally (Barr et al., Gene Therapy 1994; 1:51-58), or that the efficacy was low (White et al., Proc Natl Acad Sci 2000; 97:5428-5433; Logeart et al., Human Gene Ther 2001; 12:1601-1610) or that the application method was complicated or combined with myocardial ischemia (Boekstegers et al., Gene Therapy 2000; 7:232-240) thus not allowing to study gene transfer into a model of non-ischemic heart failure.

Trans-coronary delivery of recombinant adenovirus to rabbit myocardium.

White Zealand rabbits (weight: 3.6±0.3 kgs) received medetomidin (100μg/kg), then propofol (5 mg/kg/h) and a bolus of fentanyl (10 μg/kg). They were intubated, ventilated, ECG-, echo- and pressure-monitored throughout the experiment. Via a 5-French sheath in the carotid artery (Avanti®, Cordis, 5F, 402-605X), a JR-5-French human diagnostic catheter (Cordis; Judkins right 4.0, 5F, softouch, M538840) was placed opposite the left coronary ostium. Then, a 2.4-French Microferret (Cook) catheter was selectively introduced through the internal lumen of the first catheter into the proximal circumflex artery. The blood circulation was stopped by closing the ostium with the angiographic catheter and the left coronary artery system was first flushed with saline (2 -3 ml), then the virus supension (0.6-0.8 ml, $1*10^{10}$ pfu) was applied through the infusion catheter by using high pressure. After final flushing of the infusion catheter with saline (1-1.5 ml) the catheters were removed. No signs of ischemic damage were registered after the intervention (continuous ECG recording) or at necropsy. The cervical wound is sutured by using Vicryl® (3-0) and Nylon® (3-0). Post-operative analgesia: The animals obtained buprenorphin (Temgesic®, Boehringer, 0.01 mg /kg every 12 hours) after the operation for 72 hours.

Aortic cross-clamping

White Zealand rabbits (weight: 3.6±0.3 kgs) were investigated.

Anaesthesia:

The animals were anaesthetised with Propofol (Propofol, 2%, Fresenius) and Fentanyl (Fentanyl-Jannssen 0.5 mg, Janssen-Cilag, 0,01 mg /kg) and intubated.

Cross-clamping technique:

After preparation of the A. carotis communis dextra. a single-lumen polyurethane catheter (Cavafix®, 1.1* 1.7 mm/16G, REF 4173589, Fa. B. Braun Melsungen AG, Melsungen, Germany) was introduced into the artery. After cannulation of the A. carotis communis dextra, the thorax was opened through the third intercostal region. After opening the pericardium, a ligature (Vicryl®, 3-0) was positioned with a Deschamp arround the Aorta and A. pulmonalis. Under X-ray guidance, the tip of the catheter was fixed in the aortic bulb. After reaching the right position, the blood circulation in the heart was stopped by closing the Aorta and the A. pulmonalis with the ligature (cross-clamping). Then the catheter was flushed with saline (1-2 ml) and the virus suspension (2-3 ml, $4*10^{10}$ pfu) was applied. Finally, the catheter was flushed with saline again. After the virus application the blood flow was restored. The whole clamping process typically lasted for 10 to 15 seconds. After removing the ligature and the catheter the heart was repositioned in the pericardium. The thorax was closed by two single ligatures (Nylon®, 2-0 USP) arround the ribs and the wounds were sutured by using Vicryl® (3-0) and Nylon® (3-0).

Post-operative analgesia: The animals obtained carprofen (Rimadyl®, Pfizer, 4 mg/kg every 12 hours) and buprenorphin (Temgesic ®, Boehringer, 0.01 mg /kg every 12 hours) after the operation for 72 hours.

Model of heart failure

Medtronic pacemakers were implanted into New Zealand White Rabbits (from Harlan, Munich, Germany). Two days afterwards, rapid pacing was initiated at 320 beats/min. With this protocol, a tachycardiainduced heart failure develops reproducibly within one week. Pacing was then continued at 360 beats/min, which led to a further deterioriation of heart failure. After two weeks, the average contractility was $2200\pm320$ mmHg/sec in failing hearts and $4000\pm390$ mmHg/sec in healthy controls (p<0.05). LVEDP increased from $3.6\pm0.4$ mmHg to $13.5 \pm 1.2$ mmHg (p<0.05)

Myocardial contractility measurement by echocardiography and intraventricular tip catheterization. Left ventricular contractility was examined before and at different times after adenoviral gene transfer. The rabbits were anaesthesized as described before. Echocardiographic M mode recording was carried out as described in previous studies. Additionally, ECG and blood pressure were monitored continuously. After preparation of the right carotid artery, a Millar 2.5 F tip catheter connected to a differentiating device (Hugo Sachs, Freiburg, Germany) was placed in the left ventricle. After definition of basal contractility and LV pressure, $200\mu L$ of NaCl (0,9%) were injected as a negative control. After a sufficient equilibration period, isoproterenol was injected in doses of 0.1 to 10 $10^{-6}$ g/kg body weight. Measurements were carried out 1 minute after each injection.

Preparation and measurement of adult ventricular cardiomyocytes.

Single calcium-tolerant ventricular cardiomyocytes were prepared from myocardium after adenoviral gene transfer to rabbit hearts *in vivo.* The hearts were excised, suspended in a Langendorff apparatus and perfused retrogradely. Collagenase type II (Cell Systems, St Katharinen, Germany; 1.6 mg/ml) was infused in the presence of 0.04 mmol/L $CaCl_2$. After 15-20 minutes, the softened tissue was cut with scissors, resuspended in carbogen-gassed Powell medium (composition in mmol/L: NaCl 110, KCl 2.5, $KH_2PO_4$ 1.2, $MgS0_4$1.2, $NaCO_3$ 25, glucose 11; pH 7.4) and filtered. Increasing concentrations of $CaCl_2$ were added slowly. Finally, the cells were layered on top of Powell medium containing bovine serum albumin (40 mg/ml) and 1 mmol/L $CaCl_2$, and were allowed to sediment.

Isolated cardiomyocytes were cultured in M199 medium (supplemented with MEM vitamins, MEM non-essential aminoacids, 25 mmol/L HEPES, 10 µg/mL insulin, 100 IU/mL penicillin, 100 µg/mL streptomycin and 100 µg/mL gentamicin), on laminin-precoated dishes (5-10 µg/cm$^2$) at a density of $10^5$ cells per cm$^2$ (at 5% $CO_2$ and 37°C). The contraction amplitude of cardiomyocytes was determined using an electro-optical monitoring system with automated video edge detection (SI Systems, Heidelberg, Germany).

For fluorescence-activated cell sorting (FACS), the cells were resuspended in phosphate-buffered saline (PBS) and analyzed by flow cytometry at 520 nm for the expression of fluorescent reporter genes by using a FASCalibur with CellQuest software (Becton Dickinson, Heidelberg, Germany).

Radioligand binding for receptor transgenes.

The rabbit hearts which had been examined physiologically *in vivo* were excised and cut to pieces, resuspended in 5 mmol/L Tris-HCl, pH 7,4, 2 mmol/L EDTA and homogenized. The homogenate was centrifuged at 1000 x g for 15 min, and the supernatant was centrifuged twice at 100,000 x g for 30 min. The resulting membrane pellet was resuspended in Tris-HCl-buffer, pH 7,4. The radioligand binding experiment was carried out with varying concentrations of radioligand, incubation for an hour and subsequent filtration via Whatman C filters.

ß-Galactosidase expression.

Cardiomyocytes infected with Ad-ßGal were fixed and stained with a standard solution containing X-gal (Roche Diagnostics, Mannheim, Gemrany). Frozen hearts were cut into slices with a thickness of 7 µm in a freeze microtome, stained similarly and counterstained by hematoxylin-eosin.

Fluorescence in freeze-cut slices to study the expression of fluoresent reporter genes

Hearts were freeze-cut and expression of fluorescent reporter proteins was assessed under fluorescent light at 488 nm.

Immunofluorescence in heart slices

The slices were fixed with 3.7% formaldehyde in PBS for 20 min at room temperature and washed twice with PBS. The slices were then permeabilized with 100% methanol at -20°C (on a dry-ice block) for 30 min, or with 0.1% saponine in PBS for 30 min at room temperature and washed twice with PBS. The slices were incubated with culture medium containing 10% FCS over 30 min at 37°C (to avoid unspecific antibody binding and to reduce the background) and again washed with PBS.

The slices were then incubated for 2-3 h at 37°C with first monoclonal mouse antibody (anti fluorescing reporter gene), opportunely diluted in PBS containing 0.1 % saponine and washed three times with PBS. Then, they were incubated for 1 h at 37°C with secondary antibody (Texas Red-conjugated anti-mouse IgG, Molecular Probes), diluted 1:500 in PBS containing 0.1% saponine and washed three times with PBS. Coverslips were mounted carefully upside down on microscope slides using 1-2 drops of fluorescence mounting medium (DAKO). The slides were stored at dark at 4°C for later analysis.

Gene transfer to the vessel wall in vivo.

For gene transfer to the carotid artery, a cervical midline incision was made and the left common artery was exposed. A segment of 4 cm was isolated with two small atraumatic clips (BIEMER vessel clips, FD 561 R). Into the isolated segment, the virus solution (titer $1x10^{10}$ pfu), approximately 0,2 ml, was injected by a small needle (0,4x20 mm). The incubation time was 20 min. Then the clips were removed and the blood flow restored. The cervical wound was sutured and the animals were allowed to recover. The rabbit obtained analgesia (Buprenorphin 0,01 mg/kg sc. every 12 h) for 72 hours post operation.

Animal model and measurements.

New Zealand White rabbits were fed with a standardized chow enriched with 2% cholesterol for eight to twelve weeks. After that period, arteriosclerosis and plaques reproducibly developed in the aortic arch, the carotid arteries, the abdominal aorta and the femoral arteries of all animals. Ultrasound measurements were carried out with a General Electric Vivid-5 system connected to a 12.5 MHz ultrasound probe. The lumen width, intima-media thickness, systolic and diastolic flow velocities were investigated at standardized localisations in the carotid artery. Vessels were taken out and fixed with 4% formalin. Plaque extension was meaured in hematoxylin-eosin stained slices and on Sudan oil-stained macroscopic preparations.

Data analysis.

Data represent the mean ± standard deviation, or, where appropriate, ± standard error of the mean (SEM). Data were compared by analysis of variance for repeated measurements comparing equal doses in all groups, followed by a post-hoc Scheffe's test or by Student's t-test with two-tailed distribution where appropriate.

**Examples**

Bioreactor culture

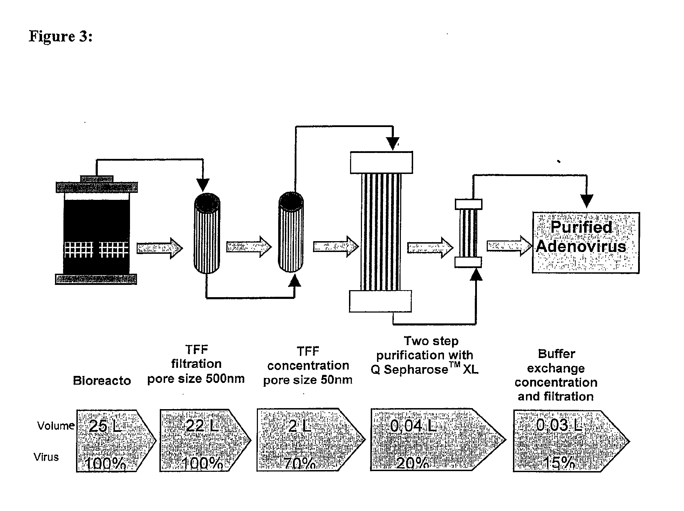

Since a packed bed reactor was chosen, cell growth can only be monitored by measuring indirect parameters, such as glucose consumption, lactate formation and oxygen-uptake rate during the cultivation process. Perfusion of the system is started when lactate concentrations in the culture exceed 1.5g/L but latest 48 h after inocculation. During the first days of virus production, lactate concentrations usually rise above 3-4 g/L indicating a starting cytopathic effect in the culture, which can simultaneously be observed by microscopy of the samples. Only if the glucose concentrations in this phase fall below 1g/L, perfusion is increased to 4-5L/d. A typical example of all culture parameters in one process is shown in figure 2, Figure 3 gives a schematic overview of the complete down-stream processing.

Total volume yields ranged from 15-22L supernatant, which can be concentrated 10-fold by tangential-flow filtration to 1-2 L. High titers of adenoviruses are susceptible to precipitation and loss of active titer, if stored at 4°C. To achieve maximum stability of the active virions, the concentrate has to be proceeded to the purification step 24-48 h after concentration. Table 1 gives typical yields of the filtration and concentration steps of the down-stream process.

Table 1:

| | Volume and Virus yields of the filtration and concentration step of a representative bioreactor batch. **1** Bioreactor, **2**: Filtration, **2.1**.: Filtrate 500nm, Membrapure Module 4000cm$^2$ PES, **2.2**: Retentate 500nm Membrapure Module 4000cm$^2$ PES, **3**: Concentration, **3.1**.: Retentate 50nm Membrapure Module 3900cm$^2$ PS, **3.2**: Filtrate 50nm Membrapure Module 3900cm$^2$ PS | | | | |
|---|---|---|---|---|---|
| | Volume (ml) | Titer (pfu/ml) | Titer (pfu) | Yield (%) | cumulative yield (%of starting titer) |
| 1 | 21.400 | 1,66x10$^8$ | 3,55x10$^{12}$ | 100 | |
| 2.1 | 21.500 | 1,66x10$^8$ | 3,6x10$^{12}$ | 100 | |
| 2.2 | 0 | - | - | - | - |
| 3.1. | 1.400 | 1,66x10$^9$ | 2,3x10$^{12}$ | 66 | |
| 3.2. | 20.000 | 2,1*10$^6$ | 4,2*10$^{10}$ | 1,2 | |
| | | | | | |

Purification of the filtered and concentrated cell supernatant

Chromatographs

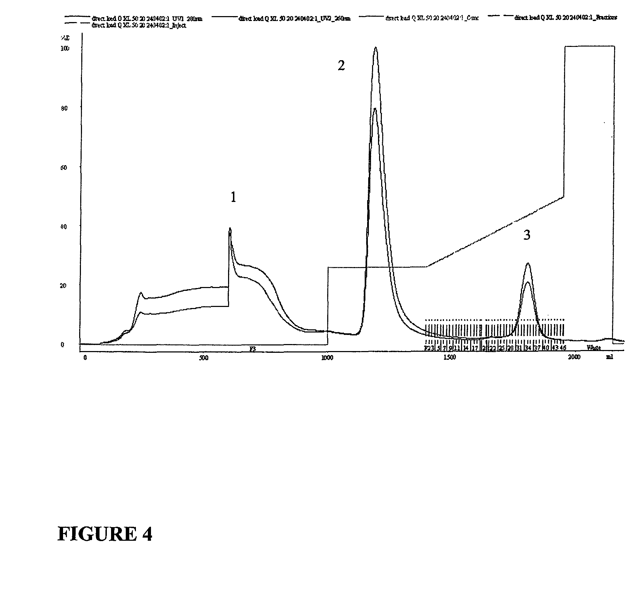

Figure 4 shows the original registration of the eluate after the first chromatography. It can be seen that the wash peak (1), the contaminant peak (2) and the adenovirus peak (3) are eluted independently of each other with a high resolution. The dotted line indicates the conductivity curve.

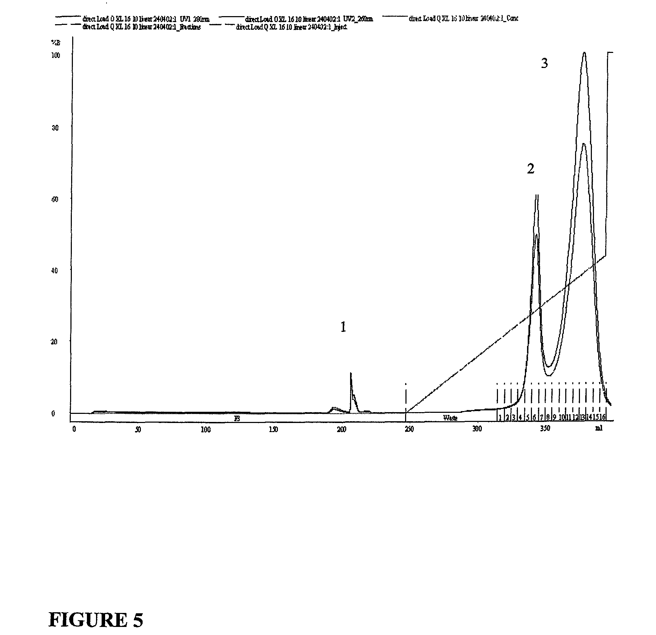

Figure 5 shows the original registration of the eluate after the second chromatography. It can be seen that the remaining contaminants are eluted in the beginning of the gradient while the adenovirus peak eluates at a higher salt concentration with a high purity (peak 2).

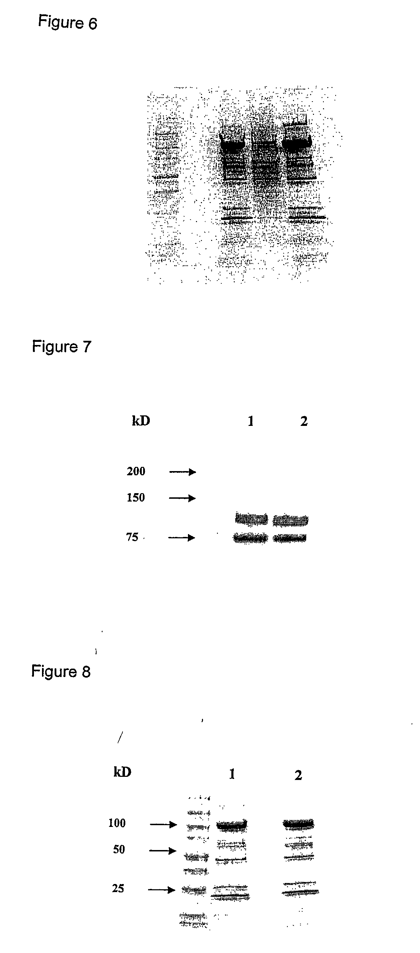

Figure 6 shows a direct comparison of the conventional one-step HPLC chromatography with the new method of double-step FPLC purification. It demonstrates the markedly higher preparation purity after two purification steps.

Virus yield

Virus yield is calculated by measuring the active titer of the in process samples and the end product in a cell-based infectivity assay. For Results, see Table 2. It can be seen that the new purification protocol resulted in a high efficiency of virus yield.

Table 2.

| Virus yield is calculated by measuring the active titer of the in process samples and the end product in a cell-based infectivity assay. The various steps in purifying the cell supernatant cause a cumulative loss of virus, typical yield of the end product being in the range of 40% like in this example. | | | | |
|---|---|---|---|---|
| sample | volume (ml) | titer (PFU/ml) | particles | yield (%) |
| **bioreactor sample filtrated and concentrated** | 700 | 1.7 x 10$^9$ | 1.2 x 10$^{12}$ | 100 |
| **flow through (XK 50/20 column)** | 700 | < 1 x 10$^4$ | < 7 x 10$^6$ | < 1 |
| **wash step (XK 50/20 column)** | 400 | ≥ 2.1 x 10$^7$ | ≥ 8.4 x 10$^9$ | ≥ 0.7 |
| **peak 1 (XK 50/20 column) at 30%B** | 400 | ≥ 2.1 x 10$^8$ | ≥ 8.4 x 10$^{10}$ | ≥ 7 |

Table 2. (continued)

| | | | | |
|---|---|---|---|---|
| Virus yield is calculated by measuring the active titer of the in process samples and the end product in a cell-based infectivity assay. The various steps in purifying the cell supernatant cause a cumulative loss of virus, typical yield of the end product being in the range of 40% like in this example. | | | | |
| sample | volume (ml) | titer (PFU/ml) | particles | yield (%) |
| peak 2 (XK 50/20 column) at 52%B diluted to 0,14 M NaCl | 244 | $3.3 \times 10^9$ | $8.1 \times 10^{11}$ | 68 |
| flow through (XK 16/10 column) | 220 | $\geq 2.1 \times 10^7$ | $\geq 4.6 \times 10^9$ | $\geq 0.3$ |
| wash step (XK 16/10 column) | 40 | $\geq 2.1 \times 10^7$ | $\geq 8.4 \times 10^8$ | $\geq 0.1$ |
| virus peak (XK 16/10 column) | 30 | $2.1 \times 10^{10}$ | $6.3 \times 10^{11}$ | 53 |
| peak S-300 | 30 | $2.1 \times 10^{10}$ | $6.3 \times 10^{11}$ | 53 |
| final dosage form | 25 | $1.9 \times 10^{10}$ | $4.8 \times 10^{11}$ | 40 |

Identity - Western blot

Fig. 7 shows a typical Western blot for adenoviral hexone proteins of two different preparations. There was no obvious difference between samples purified by the new protocol compared to cesium chloride density centrifugation.

Purity

Gel electrophoresis

Fig 8. shows a typical Coomassie-stained gel of two different adenoviral samples. There was no obvious difference between samples purified by the new protocol compared to cesium chloride density centrifugation.

Free DNA concentration

The free DNA concentration in the end product after cesium chloride density gradient was similar to the concentration in the batch purified according to the new column preparation methods.

Endotoxin assay

The endotoxin concentration in the end product after cesium chloride density gradient was $21 \pm 18$ U/ml, whereas it amounted to $46\pm7$ U/ml in the sample purified according to the new method (difference not significant by t testing).

2D-Electrophoresis and Mass Spectroscopy

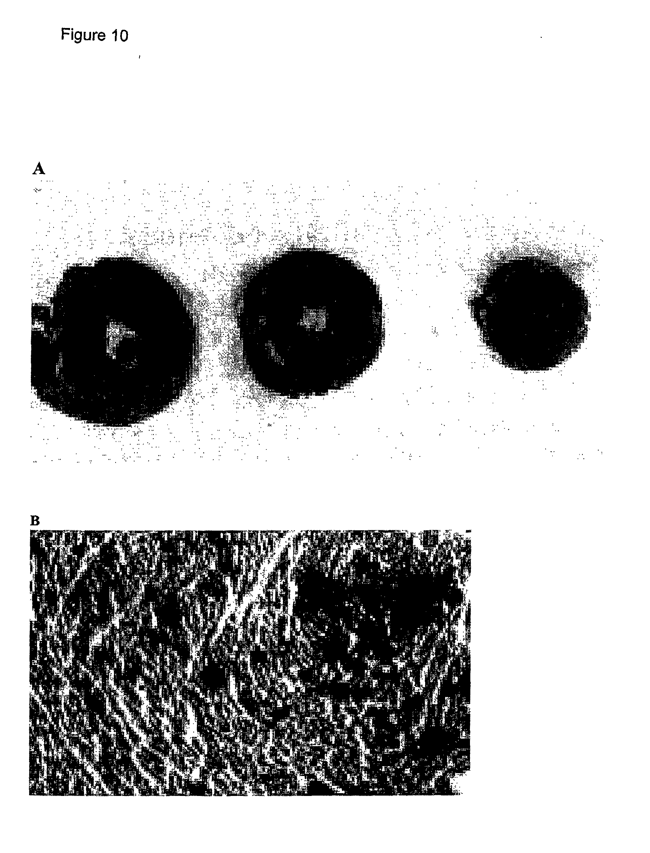

Representative preparations were analyzed for contaminant proteins by 2D gel electrophoresis (Fig. 10), and further investigated by mass spectroscopy of individual spots. Whereas adenoviral proteins were prevalent in both preparations (hexone and pentone protein subtypes) as expected, intracellular host cell proteins were additionally present in the conventional prepration (such as heat shock protein 60 and lactate dehydrogenase), which were not detectable in the sample prepared according to the new method. In both preprations, small amounts of albumin and alpha-1 antitrypsin were detected, most likely originating from additives to the host cell culture medium.

Gene transfer into the myocardium in vivo

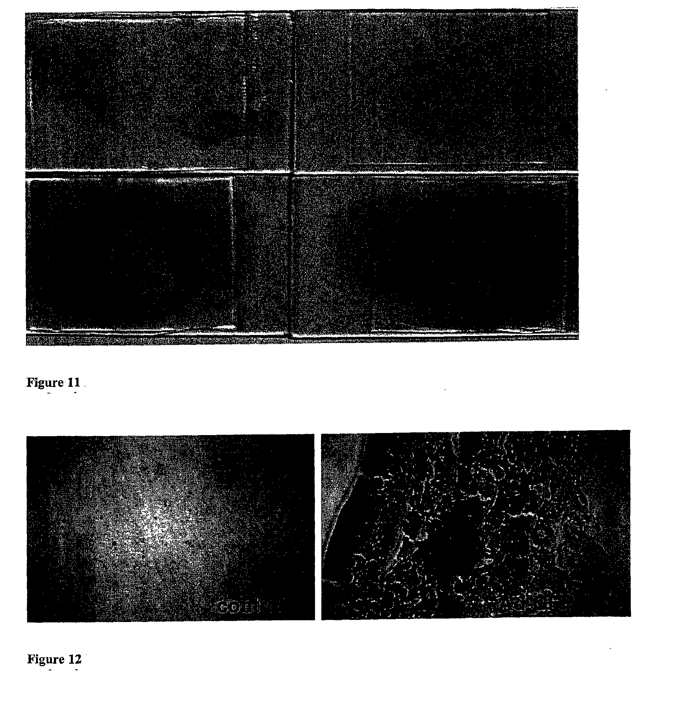

Recombinant adenovirus were used for somatic gene transfer into the myocardium. Figure 11 shows the macro- and microscopic slices after gene transfer of $10^{10}$ pfu lacZ-encoding adenovirus. A regional distribution throughout the perfusion bed of the circumflex artery can be observed macroscopically, while there is clear transgene expression on the microscopic level.

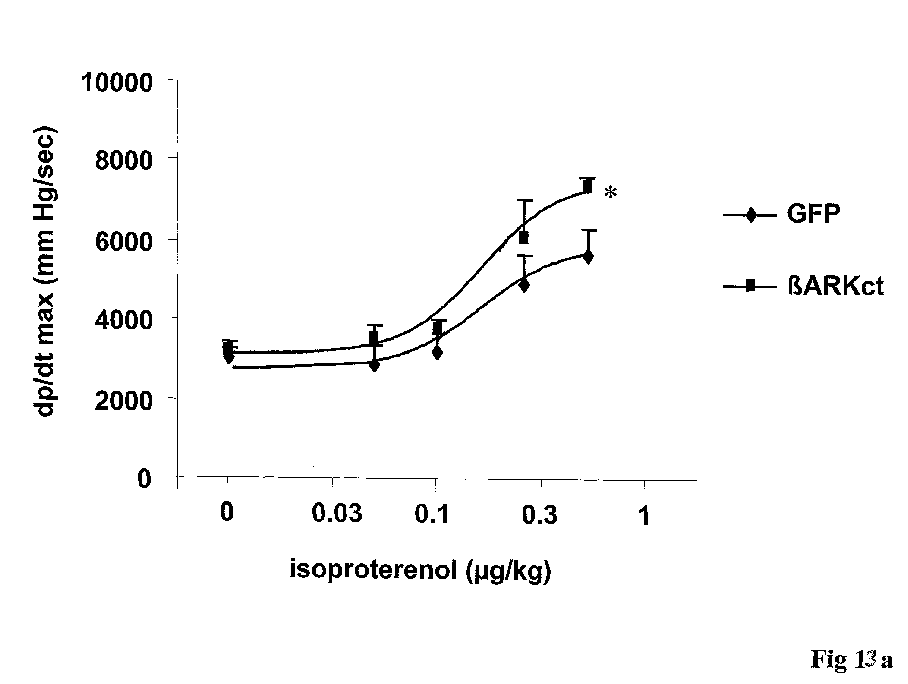

Gene transfer was also carried out by aortic cross-clamping. After gene transfer induced by high coronary pressure of 4-5 x $10^{10}$ pfu of a recombinant adenovirus encoding a reporter gene, we reliably observed widespread gene expression throughout the myocardium, as determined from paraffin-embedded slices stained with a specific antibody (Fig. 11). The Figure shows that no staining occurred after a sham intervention (Fig.11A and C). Fig 12 shows microscopic sections through infected myocardium compared to control myocardium. In a series of reproducible experiments, the whole left ventricular myocardium could be reached by a single application. However, no observable transgene expression occurred after gene transfer with the aortic cross-clamping method when using lower adenoviral titers (1 x $10^{10}$ pfu per dose and below).

As the titers necessary for the highly efficient cross-clamping protocol could only be reproducibly reached by using virus amplified at large scale from bioreactors, we compared the administration of 4 x $10^{10}$ pfu of virus batches carrying the identical transgene which were produced either from conventional cell culture wells and standard cesium chloride density gradient purification or from batches amplified in a bioreactor. The bioreactor-amplified batches were either purified with existing protocols for column purification or according to the new protocol of a double-step normal pressure FPLC column. Whereas 80% of the animals survived gene transfer with conventional preparations purified by cesium chloride and 70% of the animals with bioreactor-amplified batches purified according to the new protocol, no animal survived after gene transfer of virus samples purified with existing protocols from bioreactor batches (at least n=6 in all groups). Mortality in the latter group was due to an acute bleeding disorder and/or acute cardiovascular failure within minutes, strongly suggesting toxical elements in these virus preparations.

After using our new protocols for efficient gene transfer in vivo, we also isolated single cardiomyocytes and counted the percentage of transgene-expressing cardiomyocytes among the total number of isolated cells by fluorescence-activated cell sorting (FACS). We reproducibly found that over 30 % of the isolated cardiomyocytes express the transgene after trans-coronary gene transfer (Fig. 11). Similarly, we found that over 50% of the isolated cardiomyocytes express the transgene after high pressure gene transfer. These efficiencies were much higher than those from any other study which had so far systematically investigated the efficacy of myocardial gene transfer to larger animals in vivo (e.g., Logeart et al., Human Gene Ther 2001; 12:1601-1610, Wright et al., Gene Therapy 2001; 8:1833-1839).

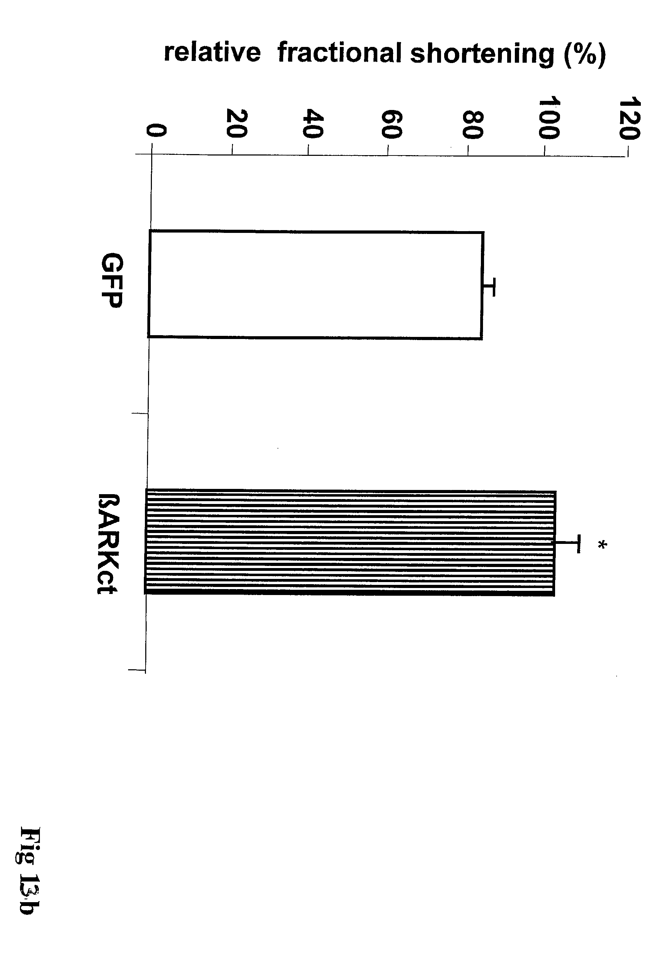

To study the functional effect of efficient myocardial gene transfer, overexpression of the transgene ßARKct in failing rabbit hearts was investigated, because it had so far been among the best studied molecular interventions with beneficial effect on heart failure, und thus served as a standard (Koch et al., Science 1995; 268:1350-1353; Harding et al.; Proc Natl Acad Sci 2001; 98:5809-5814; White et al., Proc Natl Acad Sci 2000; 97: 5428-5433). Gene transfer in vivo was carried out one week after initiation of rapid pacing and induction of heart failure. Hemodynamic investigations (echocardiography, tip catherization) were carried out one week later. There was a clear improvement of isoproterenol-induced contractility (dp/dt max; Fig 13A) as well as an improvement of echocardiographic function (intraindividual ratios of fractional shortening, Fig 13B) in the rabbits whose hearts had been infected with ßARKct-encoding adenovirus compared to the control virus-infected group.

Gene transfer to the endothelium

The carotid arteries were infected with adenoviruses encoding fluorescent reporter genes in vivo. The contralateral artery was used as a control. Fig. 15 shows a marked increase in fluorescence of the endothelial layer of the infected artery (A) compared to the sham-operated control artery (B). Gene transfer occurred throughout all the infected arterial section.

**Discussion**

The present invention provides for the first time a method for the efficient large-scale production of recombinant adenovirus in a purity suitable for *in vivo* somatic gene transfer which allows for the first time reproducible gene transfer to larger animal hearts and vessel walls in vivo.

Virus Production and Purification

For the production of adenovirus, we established standardized runs in the bioreactor which were closely monitored for several vital parameters (see Fig. 2).

So far, virtually all studies of gene transfer in vivo, and especially, to the cardiovascular system, had relied on the use of adenoviral samples prepared from cesium chloride density gradient ultracentrifugation (at 36,000-fold earth acceleration, g). Due to the limited sample volumes to be applied in ultracentrifuges, however, this method does not allow to be upscaled to the volumes needed for reproducible gene transfer to larger animal hearts and vessel walls in vivo. On the other hand, several studies had described a high pressure liquid chromatography (HPLC) system used for analytical purposes and/or for determining virus titer, which again, could not be upscaled to the handling of large volumes.

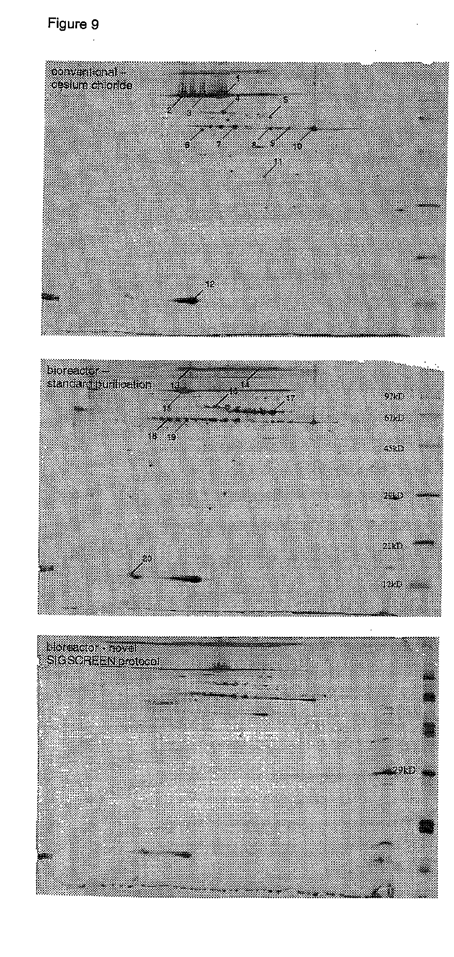

Therefore, our aim was to establish a new technology, in which the production of viruses is carried out with an novel protocol in a bioreactor. Thereafter, a tangential flow filtration is used with a special material. Finally, a novel double-step chromatography protocol was established which can be run at a pressure below 0.3 MPa (for instance, by FPLC), followed by sample dialysis at specific conditions. Taken together, this method gave the unexpected result of a very large sample size purification yielding a comparable or even better sample purity which compared well to the previous gold standard, i.e. cesium chloride density gradient ultracentrifugation (see Tables 2 and 3 and Fig. 8-10). This was evidenced by direct comparison of both methods by means of gel electrophoresis, Western blotting for viral hexone proteins, 2D gel electrophoresis and mass spectroscopy. We found that contaminations by intracellular host cell proteins (e.g., LDH and heat shock protein) were absent in the sample prepared according to the new protocol which do occur in samples prepared with the previous gold standard, cesium chloride centrifugation. As these contaminant proteins generally indicate the presence of other, highly toxic metabolites, we could be sure to have improved the purity of the viral preparations considerably. More importantly, we directly compared the tolerabilty of different virus batches after gene transfer in vivo.

Beside novel methods for production and purification of large virus samples, we have also provided a novel technology for gene transfer to the myocardium and to the vessel wall in vivo. For the first time, we could thus systematically demonstrate that the method yields a high efficiency for gene transfer into individual cardiomyocytes of larger animals in vivo (see Fig. 11-15). Previous studies had claimed some transgene expression in the myocardium, but mostly in smaller animals (e.g., Hajjar et al., Proc Natl Acad Sci 1998; 95:5251-5256), in which models of heart failure are not reliable, and they had not shown the actual percentage of cardiomyocytes which really express the transgene. In contrast, our novel method yielded a high efficiency for relevant transgene expression which also induced a functional alteration in the myocardium (see Figures 14a and b). This positive result is due both to this specific protocol with a short time high pressure administration of the virus in the aortic bulb (and not into the left ventricle as in previous publications) and to the high amounts of highly purified adenovirus which could be generated due to the novel production method. As the titers necessary for the highly efficient cross-clamping protocol could only be reproducibly reached by using virus amplified at large scale from bioreactors, we compared the administration of equal doses of virus batches which were produced either from conventional cell culture wells and standard cesium chloride density gradient purification or from batches amplified in a bioreactor. The bioreactor-amplified batches were either purified with existing protocols for column purification or according to the new protocol of a double-step normal pressure FPLC column. Whereas 80% of the animals survived gene transfer with conventional preparations purified by cesium chloride and 70% of the animals with bioreactor-amplified batches purified according to the new protocol, no animal survived after gene transfer of virus samples purified with existing protocols from bioreactor batches (at least n=6 in all groups). Mortality in the latter group was due to an acute bleeding disorder and/or acute cardiovascular failure within minutes, strongly suggesting toxical elements in these virus preparations.

Similarly, also transgene expression after gene transfer to carotid arteries occurred reliably and reproducibly after transfection with the purified virus samples in vivo.

In summary, we have established a technique for target validation in reliable large animal models of heart failure and atherosclerosis for rational, early phase drug design. Our invention solves the problem of

- production and purification of high titers of higly pure adenovirus
- using a novel FPLC-based purification protocol
- novel viral administration yielding a better expression efficiency in large animals
- thus, allowing in vivo gene expression in a clinically relevant disease background with high efficiency and low unspecific side effects or low or no mortality of the experimental animal.

**Claims**

1. A process for preparing an adenovirus-containing preparation for *in vivo* somatic gene transfer to the myocardium or endothelium of large animals, comprising the steps of

   (i) generating adenovirus in a bioreactor under predetermined conditions for obtaining an adenovirus-containing medium;
   (ii) optionally separating of adenovirus from the adenovirus-containing medium of step (i) by filtration to obtain an adenovirus-containing filtrate;

(iii) concentrating the adenovirus-containing medium of step (i) or the adenovirus-containing filtrate of step (ii) to obtain a adenovirus-containing concentrate;

(iv) batch purification of the adenovirus-containing concentrate of step (iii) by a Fast Protein Liquid Chromatography (FPLC) in at least two steps at a pressure of less than 0.3 MPa and a volume of at least 200 ml to obtain an adenovirus-containing batch;

(v) optionally concentrating the adenovirus-containing batch of step (iv) to obtain a concentrated adenovirus-containing batch; and

(vi) optionally dialysis of the adenovirus-containing batch of step (iv) or the concentrated adenovirus-containing batch of step (v),

to obtain an adenovirus-containing preparation.

2. The process of claim 1, wherein purification in step (iv) purification is conducted using columns packed with Q Sepharose XL anion exchange medium.

3. The process of claim 1 or 2 wherein subsequent to step (vi) the adenovirus-containing preparation is further processed by concentration or the addition of further components.

4. The process of claim 1, wherein the adenovirus is genetically modified.

5. The process of claim 4, wherein the virus is a recombinant replication deficient adenovirus.

6. The process of claim 4 or 5, wherein the adenovirus contains a gene of interest coding for an expression product associated with cardiovascular disease.

7. The process of any one of the preceding claims wherein in step (i) the adenovirus-containing medium is continuously harvested.

8. The process of any one of the preceding claims wherein the adenovirus is cultured in step (i) in a bioreactor containing 293 (ATCC CRL 1573) cells which are grown in high glucose DMEM supplemented with 2 percent fetal bovine serum, 2 mMN-acetyl-L-alanyl-L-glutamine, 100 mU penicillin and 100 $\mu$g/ml streptomycin at 37°C and 5 percent carbon dioxide.

9. The process of any one of the preceding claims wherein in step (ii) the adenovirus-containing medium is filtered by tangential flow filtration to obtain the adenovirus-containing filtrate.

10. The process of claim 9 wherein the pore size of the filter is in the range of 300 to 1000 nm, preferably 400 to 600 nm.

11. The process of any one of the preceding claims, wherein in step (iii) the to adenovirus-containing filtrate is concentrated by tangential flow filtration.

12. The process of claim 11, wherein the pore size of the filter is in the range of 10 to 100 nm, preferably 30 to 60 nm.

13. Method of *in vivo* validation of a target protein in a large non-human animal by somatic gene transfer of an adenovirus into the myocardium or the endothelium of a large animal, comprising the steps of

(a) providing an adenovirus-containing preparation according to the method of any one of claims 1 to 12,

(a1) wherein the adenovirus contains a gene of interest which codes for the protein to be validated and which is capable of overexpression of the protein in the myocardium or the endothelium, or

(a2) wherein the adenovirus provides for a dominant negative mutant overexpression of the protein of interest in the myocardium or the endothelium;

(b) providing a test animal as an animal model for heart and/or vessel disease;

(c) administration of the adenovirus-containing preparation to the test animal;

(d) observing the effect of the somatic gene transfer on the animal and selecting the protein as a validated protein when the presence of absence of the protein has a significant impact on the function of the myocardium or the endothelium of the animal;

whereby the animal is sacrificed in the course of the method.

14. The method of claim 13, wherein in step (c) the adenovirus-containing preparation is administered to the myocardium under high pressure in a high dosage over a short period of time.

15. The method of claim 13, wherein the animal is a rabbit.

16. The method of any one of claims 13 to 15, wherein the animal is an animal model for heart failure.

**4-GAS**

LEVEL
PROBE

$DO_t$

$DO_b$

POLYESTER
DISCS

IMPELLER
WITH
SPARGER

HARVEST
RESERVOIR

MEDIA
RESERVOIR

Figure 1:

**Figure 2:**

A

B

Figure 3:

| | Bioreacto | TFF filtration pore size 500nm | TFF concentration pore size 50nm | Two step purification with Q Sepharose™ XL | Buffer exchange concentration and filtration |
|---|---|---|---|---|---|
| Volume | 25 L | 22 L | 2 L | 0.04 L | 0.03 L |
| Virus | 100% | 100% | 70% | 20% | 15% |

**FIGURE 4**

**FIGURE 5**

Figure 6

Figure 7

Figure 8

Figure 9

Figure 10

A

B

Figure 11

Figure 12

Fig 13a

Fig 13b

Figure 14

GFP fluorescence

Figure 15

**European Patent Office**

## EUROPEAN SEARCH REPORT

Application Number

EP 02 01 3017

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,X | WO 00 40702 A (AVENTIS PHARMA SA ;BARBOT ANNE (FR); BLANCHE FRANCIS (FR); CAMERON) 13 July 2000 (2000-07-13) * page 1, paragraph 3 * * page 3, paragraph 2 - paragraph 3 * * page 4, paragraph 2 * * page 5, paragraph 4 * * page 6, line 23 - page 7, line 20 * * page 8, paragraph 3 * * page 9, paragraph 1 * * page 10, paragraph 1 * * example 7 * | 1-16 | C12N7/02 A61K48/00 |
| D,X | HUYGHE B G ET AL: "PURIFICATION OF A TYPE 5 RECOMBINANT ADENOVIRUS ENCODING HUMAN P53 BY COLUMN CHROMATOGRAPHY" HUMAN GENE THERAPY, XX, XX, vol. 6, 1 November 1995 (1995-11-01), pages 1403-1416, XP000196636 ISSN: 1043-0342 * the whole document * | 1-16 | |
| X | WO 99 54441 A (GENVEC INC) 28 October 1999 (1999-10-28) * examples 1-4 * | 1-16 | TECHNICAL FIELDS SEARCHED (Int.Cl.7) C12N |
| D,X | WO 98 22588 A (INTROGEN THERAPEUTICS INC ;CHO TOOHYON (US); ZHANG SHUYUAN (US); T) 28 May 1998 (1998-05-28) * the whole document * | 1-16 | |
| D,X | WO 98 00524 A (GUILLAUME JEAN MARC ;BLANCHE FRANCIS (FR); RHONE POULENC RORER SA) 8 January 1998 (1998-01-08) * example 5 * | 1-16 | |

-/--

The present search report has been drawn up for all claims

| Place of search | Date of completion of the search | Examiner |
|---|---|---|
| THE HAGUE | 19 November 2002 | Niemann, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03 82 (P04C01)

**European Patent Office**

**EUROPEAN SEARCH REPORT**

Application Number

EP 02 01 3017

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | ALEXANDER M Y ET AL: "Gene transfer and models of gene therapy for the myocardium." CLINICAL AND EXPERIMENTAL PHARMACOLOGY & PHYSIOLOGY. AUSTRALIA SEP 1999, vol. 26, no. 9, September 1999 (1999-09), pages 661-668, XP002221527 ISSN: 0305-1870 * the whole document * | 13-16 | |

TECHNICAL FIELDS SEARCHED (Int.Cl.7)

The present search report has been drawn up for all claims

| Place of search | Date of completion of the search | Examiner |
|---|---|---|
| THE HAGUE | 19 November 2002 | Niemann, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT
## ON EUROPEAN PATENT APPLICATION NO.

EP 02 01 3017

This annex lists the patent family members relating to the patent documents cited in the above-mentioned European search report.
The members are as contained in the European Patent Office EDP file on
The European Patent Office is in no way liable for these particulars which are merely given for the purpose of information.

19-11-2002

| Patent document cited in search report | | | Publication date | Patent family member(s) | | | Publication date |
|---|---|---|---|---|---|---|---|
| WO 0040702 | A | | 13-07-2000 | FR | 2788064 | A1 | 07-07-2000 |
| | | | | AU | 3049300 | A | 24-07-2000 |
| | | | | BR | 9916654 | A | 15-01-2002 |
| | | | | CN | 1332796 | T | 23-01-2002 |
| | | | | CZ | 20012428 | A3 | 14-11-2001 |
| | | | | EP | 1141249 | A1 | 10-10-2001 |
| | | | | WO | 0040702 | A1 | 13-07-2000 |
| | | | | HU | 0104794 | A2 | 29-04-2002 |
| | | | | NO | 20013271 | A | 28-08-2001 |
| | | | | PL | 349074 | A1 | 01-07-2002 |
| | | | | US | 2002037565 | A1 | 28-03-2002 |
| WO 9954441 | A | | 28-10-1999 | AU | 3662099 | A | 08-11-1999 |
| | | | | BG | 104928 | A | 28-09-2001 |
| | | | | BR | 9909789 | A | 26-12-2000 |
| | | | | CA | 2328462 | A1 | 28-10-1999 |
| | | | | EE | 200000605 | A | 15-04-2002 |
| | | | | EP | 1073721 | A1 | 07-02-2001 |
| | | | | HU | 0102209 | A2 | 28-09-2001 |
| | | | | JP | 2002512361 | T | 23-04-2002 |
| | | | | NO | 20005295 | A | 30-11-2000 |
| | | | | PL | 343630 | A1 | 27-08-2001 |
| | | | | SK | 15682000 | A3 | 10-05-2001 |
| | | | | WO | 9954441 | A1 | 28-10-1999 |
| | | | | US | 6383795 | B1 | 07-05-2002 |
| | | | | US | 2002034735 | A1 | 21-03-2002 |
| WO 9822588 | A | | 28-05-1998 | AU | 732703 | B2 | 26-04-2001 |
| | | | | AU | 5361798 | A | 10-06-1998 |
| | | | | BR | 9713368 | A | 18-09-2001 |
| | | | | CN | 1244215 | A | 09-02-2000 |
| | | | | EP | 0968284 | A2 | 05-01-2000 |
| | | | | JP | 2001504701 | T | 10-04-2001 |
| | | | | NO | 992389 | A | 19-07-1999 |
| | | | | NZ | 335947 | A | 22-12-2000 |
| | | | | WO | 9822588 | A2 | 28-05-1998 |
| | | | | US | 6194191 | B1 | 27-02-2001 |
| WO 9800524 | A | | 08-01-1998 | FR | 2750433 | A1 | 02-01-1998 |
| | | | | AU | 3447097 | A | 21-01-1998 |
| | | | | BR | 9710030 | A | 10-08-1999 |
| | | | | CA | 2258158 | A1 | 08-01-1998 |
| | | | | CZ | 9804383 | A3 | 17-03-1999 |
| | | | | EP | 0944717 | A1 | 29-09-1999 |
| | | | | WO | 9800524 | A1 | 08-01-1998 |
| | | | | JP | 2000514290 | T | 31-10-2000 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 02 01 3017

This annex lists the patent family members relating to the patent documents cited in the above-mentioned European search report.
The members are as contained in the European Patent Office EDP file on
The European Patent Office is in no way liable for these particulars which are merely given for the purpose of information.

19-11-2002

| Patent document cited in search report | Publication date | Patent family member(s) | | Publication date |
|---|---|---|---|---|
| WO 9800524 A | | NO | 986202 A | 15-02-1999 |
| | | SK | 181098 A3 | 12-07-1999 |
| | | US | 2002028497 A1 | 07-03-2002 |
| | | HU | 0001271 A2 | 28-08-2000 |

EPO FORM P0459

For more details about this annex : see Official Journal of the European Patent Office, No. 12/82